# EUROPEAN PATENT SPECIFICATION

(11) **EP 3 713 496 B1**
(45) Date of publication and mention of the grant of the patent: **23.07.2025**
(21) Application number: 18896747.5
(22) Date of filing: 17.12.2018
(51) Int. Cl.: A61K 41/00, A61K 47/69, A61K 47/65, A61P 35/00, A61K 9/127, A61K 9/00

(54) **ULTRASOUND SUSCEPTIBLE MAGNETIC DIRECTED NANO DRUG CARRIER SYSTEM**
ULTRASCHALLEMPFINDLICHES, MAGNETISCH GERICHTETES NANO-MEDIKAMENT-TRÄGERSYSTEM
SYSTÈME TRANSPORTEUR DE NANOMÉDICAMENT DIRIGÉ MAGNÉTIQUEMENT, SENSIBLE AUX ULTRASONS

(30) Priority: 29.12.2017 TR 201723077
(43) Date of publication of application: 30.09.2020
(73) Proprietor: Ege Universitesi, 35100 Izmir (TR)
(72) Inventor: SANLIER, Senay, Izmir (TR); AK, Güliz, Izmir (TR); YILMAZ, Habibe, Izmir (TR); YALÇIN, Murat, Bursa (TR); KINAL, Armagan, Izmir (TR); NALBANTSOY, Ayse, Izmir (TR); YILDIRIM, Yeliz, Izmir (TR)
(74) Representative: Potter Clarkson
(86) International application number: PCT/TR2018/050814
(87) International publication number: WO 2019/132830

(56) References cited:
- WO-A1-2016/134115
- WO-A1-2017/040976
- WO-A2-03/074005
- US-A1- 2011 054 236
- US-A1- 2015 259 380
- CHING-HSIANG FAN ET AL: "Ultrasound/Magnetic Targeting with SPIO-DOX-Microbubble Complex for Image-Guided Drug Delivery in Brain Tumors", THERANOSTICS, vol. 6, no. 10, 1 January 2016 (2016-01-01), AU, pages 1542 - 1556, XP055756073, ISSN: 1838-7640, DOI: 10.7150/thno.15297
- FAN CHING-HSIANG ET AL: "SPIO-conjugated, doxorubicin-loaded microbubbles for concurrent MRI and focused-ultrasound enhanced brain-tumor drug delivery", BIOMATERIALS, ELSEVIER, AMSTERDAM, NL, vol. 34, no. 14, 20 February 2013 (2013-02-20), pages 3706 - 3715, XP028985990, ISSN: 0142-9612, DOI: 10.1016/J.BIOMATERIALS.2013.01.099
- AK ET AL: "Kanser hücrelerine hedefli biyomimetik ilaç tasima sistemlerinin hazirlanmasi, karakterizasyonu ve biyopotansiyelinin arastirilmasi [Preparation and characterization of cancer cell targeted biomimetic drug carrier systems and research their biopotential]", 30 November 2015 (2015-11-30), pages 1 - 193, XP009521574, Retrieved from the Internet <URL:https://tezarsivi.com/kanser-hucrelerine-hedefli-biyomimetik-ilac-tasima-sistemlerinin-hazirlanmasi-karakterizasyonu-ve-biyopotansiyelinin-arastirilmasi>
- MANASMITA DAS ET AL: "Bio-functionalization of magnetite nanoparticles using an aminophosphonic acid coupling agent: new, ultradispersed, iron-oxide folate nanoconjugates for cancer-specific targeting", NANOTECHNOLOGY, INSTITUTE OF PHYSICS PUBLISHING, GB, vol. 19, no. 41, 15 October 2008 (2008-10-15), pages 415101, XP020144670, ISSN: 0957-4484, DOI: 10.1088/0957-4484/19/41/415101
- ZSANETT MIKLAN ET AL: "New pemetrexed-peptide conjugates: synthesis, characterization and in vitro cytostatic effect on non-small cell lung carcinoma (NCI-H358) and human leukemia (HL-60) cells", JOURNAL OF PEPTIDE SCIENCE, vol. 17, no. 12, 1 December 2011 (2011-12-01), pages 805 - 811, XP055019105, ISSN: 1075-2617, DOI: 10.1002/psc.1407
- SANLIER SENAY HAMARAT ET AL: "Development of Ultrasound-Triggered and Magnetic-Targeted Nanobubble System for Dual-Drug Delivery", JOURNAL OF PHARMACEUTICAL SCIENCES, vol. 108, no. 3, March 2019 (2019-03-01), pages 1272 - 1283, XP009524402
- AK, GÜLIZ: "Kanser hücrelerine hedefli biyomimetik ilaç taşıma sistemlerinin hazırlanması, karakterizasyonu ve biyopotansiyelinin araştırılması [Preparation and characterization of cancer cell targeted biomimetic drug carrier systems and research their biopotential]", THESIS IN BIOCHEMISTRY, 2016, Izmir, Turkey, pages 1 - 193, XP009521574
- DAS, MANASMITA ET AL.: "Bio-functionalization of magnetite nanoparticles using an aminophosphonic acid coupling agent: new, ultradispersed, iron-oxide folate nanoconjugates for cancer-specific targeting", NANOTECHNOLOGY, vol. 19, no. 41, 415101, 2008, XP020144670

## Description

### Technical Field

The invention relates to drug carrier systems that are directed to be used in treatment for lung cancer and particularly except small cell lung cancer and to a preparation method of the same.

### Background of the Invention (Prior Art)

Lung cancer accounts for 13.4 % of all types of cancer. 28.4 % of cancer-related deaths is resulted from lung cancer. Such high rate leads to need for making novel strategies in lung cancer treatment. The American Cancer Society defines the methods for cancer treatment as chemotherapy, radiotherapy, immunotherapy, hormone therapy, targeted therapy, stem cell or bone marrow transplantation and surgery. Among currently known treatment methods, targeted cancer therapy has not been commonly used yet. Additionally, targeting strategies that have already been used are quite limited.

Many chemotherapeutic agents are intravenously applied. Inhaled therapy is advantageous for lung cancer treatment as such therapy enables the chemotherapeutic agent to directly reach to malign tissues. However, many drugs are not applicable to such inhaled therapy applications.

In the present invention; a magnetic directed, ultrasound susceptible nano drug carrier system that comprises dual drug to be used in lung cancer therapies is provided. Unlike current methods, more than one targeting strategies are used in this newly developed system.

Ching-Hsiang Fan et al., Theranostics, vol. 6, no. 10, 1 January 2016, pages 1542-1556, describes ultrasound/magnetic targeting with SPIODOX-microbubble complex for image-guided drug delivery in brain tumours.

Fan Ching-Hsiang et al., Biomaterials, vol. 34, no. 14, 20 February 2013, pages 3706-3715, describes SPIO-conjugated, doxorubicin-loaded microbubbles for concurrent MRI and focused-ultrasound enhanced brain-tumour drug delivery.

Ak et al., Thesis in Biochemistry, Ege University Graduate Faculty of Natural and Applied Science, Izmir, Turkey, 30 November 2015, pages 1-193, describes preparation and characterization of cancer cell targeted bionnimetic drug carrier systems and research their biopotential.

Manasmita Das et al., Nanotechnology, vol. 19, no. 41, 15 October 2008, page 415101, describes Bio-functionalization of magnetite nanoparticles using an aminophosphonic acid coupling agent: new, ultradispersed, ironoxide folate nanoconjugates for cancer-specific targeting.

Zsanett Miklan et al., Journal Of Peptide Science, vol. 17, no. 12, 1 December 2011, pages 805-811, describes new pemetrexed-peptide conjugates: synthesis, characterization and in vitro cytostatic effect on non-small cell lung carcinoma (NCI-H358) and human leukemia (HL-60) cells.

### Brief Description and Aims of the Invention

The primary aim of the invention is to provide an alternative and efficient solution to lung cancer treatment and thus a more effective therapy. With the drug system developed in the invention, a novel solution is oferred to lung cancer treatment, and this targeted chemotherapeutic therapy has numerous advantages as follows:
- conducting a controlled drug release due to ultrasound susceptible nature of nanobubble structure of this argon gas loaded system,
- enabling this system to be only accumulated over the region on which it is anticipated to have an effect thanks to magnetic targeting feature,
- that this system comprising dual drug has a peptide sequence that can be specifically disintegrated by matrix metallaproteinase-11 having an increased expression rate in lung cancer, and that is conjugated with magnetic nanoparticles,
- that, in this developed system, the chemotherapeutics called pemetrexed and pazopanib can be dually used and thus that more amount of cytotoxic can be obtained in cancer cells due to a combined application of pemetrexed antifolate effect and pazopanib tyrosine kinase inhibition effect,
- with the present invention, that a subject can be enabled to have more advantages due to low dose drug with an increase in therapeutic index of drugs used in treatment by way of controlled drug release and magnetic targeting,
- ensuring an improved well-being for the subject and also a lower cost,
- Obtaining a decrease in toxicity matter in a healthy tissue with directing the system developed to a target tumor tissue, and at the same time, an increase in therapeutic effect of drugs,
- enabling the nano structure obtained by means of the lipid bilayer layer-formed structure of the system developed to be easily penetrated into the tumor tissue,
- applying this system in two different ways including both in intravenous manner and also in inhaled manner to the subject,
- allowing the system to be only accumulated over the region on which it is anticipated to have an effect thanks to magnetic targeting.

### Definitions of the Drawings of the Invention

**Figure1****:** SEM view of magnetic nanoparticles
**Figure2****:** HPLC analysis result of Pemetrexed
**Figure3****:** HPLC analysis result of Pem(OMe₂)
**Figure4****:HPLC** analysis result of CIAc-Pem(OMe₂)
**Figure5****:** HPLC analysis result of Pazopanib
**Figure6****:** HPLC analysis result of CIAc-Pz
**Figure7****:** FTIR spectrum of MNP-PPP
**Figure8****:** C-TEM images of (A)NB-800and(B)NB-400 samples,
**Figure9****:** % cell vitality data: A)MNP-PPPB) Liposome C) Nanobubble obtained as a result of a 48-hour incubation of A549-luc,HTB177,CRL5807,CRL5826andCCD-34Lu cell lines with drug groups at variable doses (12,5-250µg/mL).

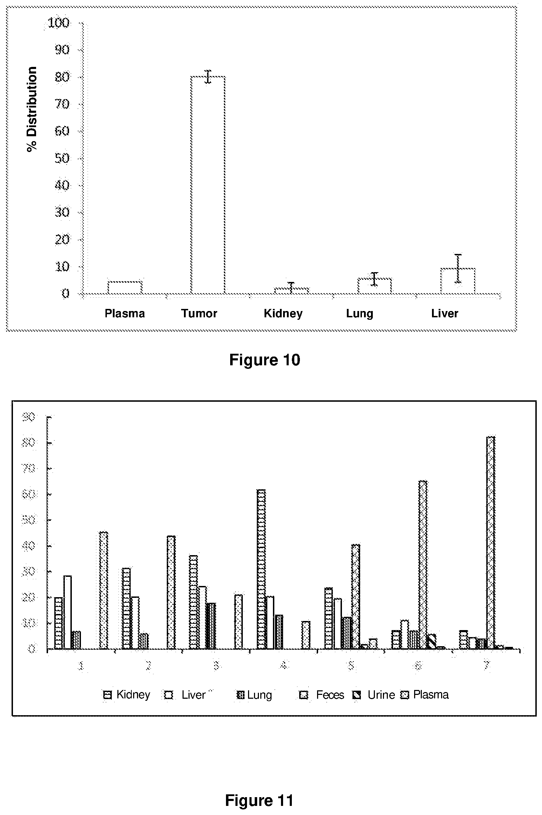

**Figure10****:** Nanobubble distribution percentages.
**Figure11****:** Nanobubble distribution in organs at determined times.

### Detailed Description of the Invention

The invention relates to the ultrasound susceptible magnetic directed nano drug carrier system designed to be used in lung cancer treatment. Magnetic nanoparticles are synthesized based on iron salts for magnetic targeting. A hexapeptide capable of disintegrating enzyme matrix metalloproteinase-11 which is not available in healthy tissue but increases in solid lung tumors has been designed in an attempt to enable the system to release drug more selectively in lung cancers, and also is covalently bound to a surface of the magnetic nanoparticle. The drugs are pemetrexed and pazopanib.

Many scientists commonly agree on the idea that inhibition of only one pathway in cancer treatment would not yield fruitful outcomes that much. Therefore, in the drug carrier system subject to the present invention, the drugs that are routinely used in clinics are administered in combined manner. For this reason, two different chemotherapy agents, pemetrexed and pazopanib, are loaded to magnetic peptide nanoparticles for ensuring a dual therapy. Such dual use; when administered by combining antifolate effect of the pemetrexed and tyrosine kinase inhibition effect of the pazopanib, allows obtaining more amount of cytotoxic in cancer cells. Bondingbetween peptide and drugs is provided through thiol group of cysteine.

Magnetic conjugate obtained is retained within a lipid-structured liposome in order to enable the same to circumvent the circulation. The liposome; is formed using 1,2-dipalmitoil-sn- glisero-3-fosfokolin(DPPC),1,2-dioleil-sn-glisero-3-fosfokolin(DOPC)and cholesterol, and is subsequently passed through membranes via an extruder and thereby made smaller in size. Following the size reduction process, chemically inert and biocompatible argon (Ar) gas is loaded into the liposome, which makes the later ultrasound susceptible, and thus bubble structure is formed. For increasing stability, the system is made ready by conducting a freezing and thawing process on dry ice. The ultrasound susceptible magnetic directed nano drug carrier system is directed to the tumor tissue with a magnetic area that is locally applied from the outside, and after targeting process, a nanoconjugate structure synthesized with the ultrasound that is locally applied is extruded from the nanobubble and thus enabled to accumulate on said region.

Biocompatibility of the system is tested based on bounding to serum proteins, haemolysis amount, and phagocytosis detection by macrophage cells, and also parameters such as determination of cytotoxicity and apoptosis effect are determined with cell culture experiments. In vivo tests are conducted by forming a tumor model with injection of the except small cell lung cancer line. Single dose and repeated dose toxicityexperiments, biodistribution and pharmacokinetics tests are made, in vivo tests are carried out and thus it is determined that the system is directed to the target tissue according to its aim and is capable of providing a therapeutic effect by being released in controlled manner over this region.

In the drug carrier system subject to the present invention; easier targeting with magnetic targeting, controlled drug release with ultrasound application, and specific targeting with matrix metalloproteinase-11 is provided.

A method for preparing the ultrasound susceptible magnetic directed nano drug carrier system to be used in cancer treatment, particularly except small cell lung cancer treatment comprises the following process steps:
i)synthesizing magnetic nanoparticles,
ii)aminating synthesized magnetic nanoparticles,
iii)modifying drugs
iv)conjugating each modified drug with separate peptides,
v)obtaining nanoconjugates by bonding aminated magnetic nanoparticles with peptide-drug conjugates,
vi) synthesizing nanoconjugate-loaded nanobubbles.

### Magnetic Nanoparticle Synthesis

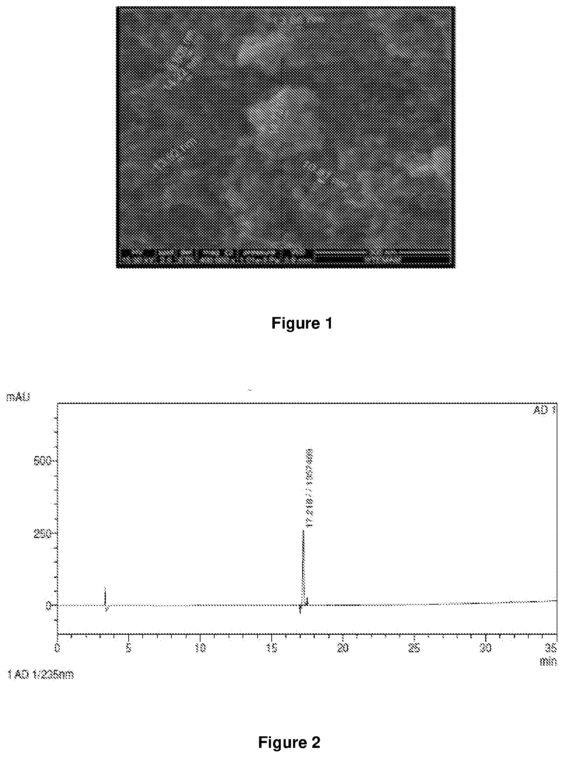

Magnetic nanoparticles (MNP) are initiallysynthesized using the method of coprecipitation in order to obtain the magnetic nanoparticles carrying an active amine group. In the present invention, preferably 0.5-2 moles of iron (II) chloride (FeCl2) and 1-4 moles of iron (III) chloride (FeCl3) are dispersed in deoxygenated distilled water within a jacketed reactor at a constant temperature of 50-70°C, preferably 60°Cand at 3000-5000 rpm mixing speed. During this process, nitrogen (N₂) gas is continuously passed through the medium. Subsequently, with addition of concentrated base solution, preferably concentrated ammonium hydroxide (NH₄(OH)₂) solution as precipitation agent to the solution, pH of the medium is adjusted to alkali (pH 9-11). The solution is left to 1-3-hour incubation for nucleation under the constant temperature (50-70°C) and powerful mixing. The nanoparticles formed at the end of the duration are separated with magnetic decantationand subsequently washed with distilled water and ethanol (EtOH). Washed nanoparticles are dried nightlong in an incubator at the temperature of 50-70°C, preferably 60°C, and thus MNP crystals are obtained. Scanning electron microscope (SEM) image as to MNP samples is given in Figure 1.

Numerous methods are provided for magnetite synthesis. The method used has a direct effect on character of the magnetite obtained. Coprecipitation method is preferred in the invention with regard to proper shape, size, magnetic power and simple applicability of the method.

Ammonium hydroxide is used as a precipitation agent in synthesis of magnetic nanoparticles by use of copresipitation method. Basic character of the ammonium hydroxide enables the medium to be alkali.

Oxygen is removed from the medium by passing nitrogen gas through the same in the invention. Removing oxygen from the medium is important for preventing oxidation to different derivatives of the magnetite. In addition, size of the magnetic nanoparticles generated in an oxygen-free medium is much smaller.

Magnetic nanoparticles are synthesized with various bases under alkali conditions. The reaction occurring when magnetic nanoparticle is formed is as the following;

Fe⁺² + 2Fe⁺³ + 8 OH⁻ Fe₃O₄ + 4H₂O

As can be seen in the reaction, the mole ratio of 1:2 is significant in terms of magnetic nanoparticle formation.

In the invention, experiments are conducted preferably at the temperature of 60°C in order to allow the reaction to occur.

A high speed is required in order to ensure crystallization and solid catalyzed reaction. As a higher speed allows smaller and homogeneous nanoparticle formation, higher speeds are preferred for the experiments conducted in the invention.

### Magnetic Nanoparticle Synthesis Carrying Amine Group

2-Aminoethylphosphonic acid (APA) solution is added to the medium comprising magnetic particle and having a pH of 5-7. Subsequently, it is left to incubation for a reaction at 70-90°C and 1500-3000 rpm. At the end of the duration, the MNPs carrying the active amine group are washed with distilled water with the help of magnetic decantation and made ready to disperse in phosphate-salt-buffer (PBS).

### Modification of Pemetrexed

Prior to conjugation of the Pemetrexed drug with peptide, a modification experiment with the Pemetrexed is conducted and the drug is enabled to be chloro-acetylated. Therefore, two-step modification is applied. In the first step, methoxylation and in the second stepchloracetylenationis performed. In the first modification; 100-200 mmol of thionyl chloride (SOCl₂) is slowly added to cold 5-15 ml of methanol (MeOH) at -15-(-5)°C and subsequently left to reaction at -10°C for 20 minutes. At the end of the duration, the temperature of the reaction medium is increased to 20-30 °C by adding 50-150 mg of pemetrexedin sodium salt, and incubated for 2-4 hours by being mixed under a condenser. Sodium chloride (NaCl) formed following the reaction is separated with filtration and evaporated out with methanol vacuum.

The excess of the compound SOCl₂ is removed by being successivelydissolved in hot methanol. The product obtained is crystallized in ethanol and the crystals formed are separated from the medium by filtration, and dried by being treated with diethylether ((C₂H₅)₂O). Pem (OMe)₂ crystals in light green colorare obtainedas a result of the first-step modification of the Pemetrexed.

After completing this first step, chloroacetic acid (ClCH₂CO₂H) group is formed in the methoxylatedPemetrexed. In the second-step modification, Pem(OMe)₂crystals are dissolved within 0,3-0,5 ml of dimethyl formamide (DMF). Subsequently, 7-15µl of diisopropylethylamine and 0.10-0.20 mg of chloroacetic anhydride are added. This medium is left to reaction for 4-6 hours at the temperature of 30-50°C. At the end of the duration, acetonitrile (ACN): distilled water (containing 0.05-0.15% of trifluoroacetic acid (TFA)) and 0.1-0.3 ml of distilled water at the ratio of 80%:20-70%:30 (v/v) are added to the mixture and it is left to incubation for 10-20 hours at room temperature. Following the incubation, the yield is precipitated by adding distilled water to the medium and removed from the medium by means of centrifuge method. The yield is crystallized in ethanol and left to dry after being treated with dietylether. Consequently, chloroacetylated permetrexed (ClAc-PEM (OMe₂)) yield is obtained. HPLC chromatograms as to Pemetrexed and modified species are given in Figures 2, 3 and 4.

Pemetrexed shows activity over the carboxyl (-COOH) group available in its structure.The pemetrexed needs to be chloroacetylated to be able to be conjugated with peptide over thiol group. Not to conduct chloroacetylation over the -COOH groups, these groups are initially preserved by being methoxylated. This is the first-step modification. Subsequently, with the second-step modification, the chloroacetyl group through which it would be bonded to the peptide is formed.

### Modification of Pazopanib

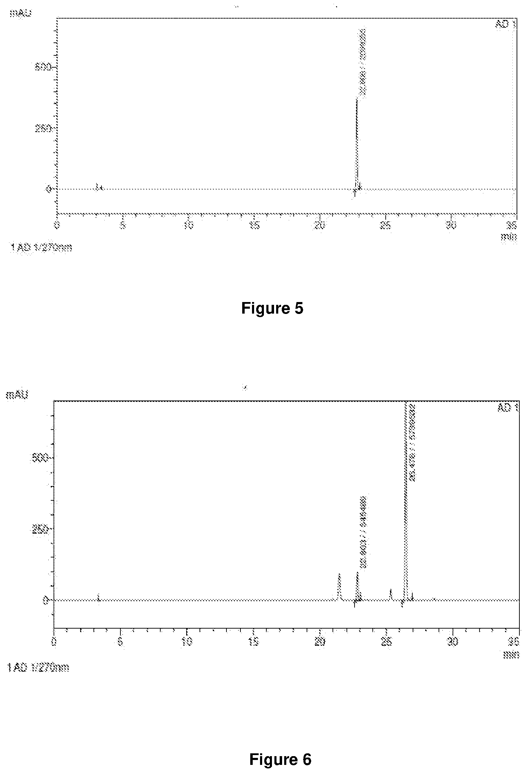

In modification experiment of the pazopanib, the drug is converted to reactive form by bonding the chloroacetate with the method proposed in chloroacetylation reaction carried out in the second step of the pemetrexed modification to the amine (NH₂) group bonded to sulfur dioxide (SO₂) available on the structure. The first step of the the modification method proposed for the pemetrexed in the pazopanib modification is skipped and only the second step is applied. HPLC chromatograms as to the pazopanib and its modified state are given in Figures 5 and 6.

The pazopanib is bonded to peptide over thiol group. As it has no functional group that needs to be preserved, the only modification in the 2nd step of pemetrexed modificationis conducted in order to form the chloroacetyl group through which it would be bonded to the peptide.

### HPLC Analysis

With reference to areas of peaks of the Pemetrexed and Pem(OM₂); it is discovered that the sample analyzed in calculations conducted in the equation of standard graphic has 1,93 ppm of free pemetrexed and 128,87 ppm of Pem (OMe₂). According to this result, it is seen that the first-step modification yield of the drug is 98.5%.

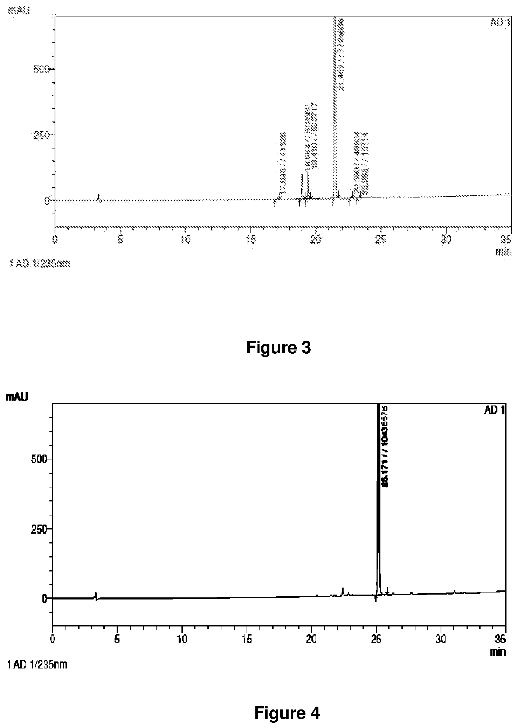

In the sample introduced to the HPLC analysis following the second-step modification of the drug Pemetrexed, peak of the Pem (OMe₂) is observed, which is a free drug and a yield of the first modification. It is seen that the retention time in the yield analyzed is 27,171 and as a result of the calculation carried out from the area of this peak, ClAc-Pem(OMe2) is found, which is a second modification yield of 173.62 ppm. As seen from Figure 4, as the peak of the free and first modified drug is not observed, it is determined that the second modification of the drug occurred 100%.

According to the calculations conducted from peak areas of the Pazopanib, it is found that the samplehas 7,786 ppm of free pazopanib and 72,72 ppm of modified pazopanib. When having yield calculation made, it is seen that the modification of the drug Pazopanib occurred at the rate of 90.34%.

### Conjugation of Drugs with Peptide

The peptide structure designed for the aim is like Ala-Lys-Ala-Leu-Arg-Cys. The modified pemetrexed and pazopanib are bonded over the thiol group of cysteine amino acid of peptide with application of the same method. In the peptide-drug conjugation, the reaction between the drug and the peptide involving cysteine, tris buffer: DMF (45:55 - 40:60, %v/v) solvent mixture is used. Initially, 0,01-0,05 mmol of modified drug is dissolved in 7-15 ml of DMF and subsequently, 5-15 ml 0.05-0.2 M tris buffer (pH 7-8) is added to the medium. Then, 0.005-0.025 mmol of peptide is slowly added to the reaction medium. The samples are left to incubation for a duration 15-25 hours in a shaking incubator at room temperature. At the end of the period, the solvent is removed and peptide-drug conjugations are obtained.

### Bonding Peptide-Drug Conjugations to Magnetic Nanoparticles Carrying Active Amine Group

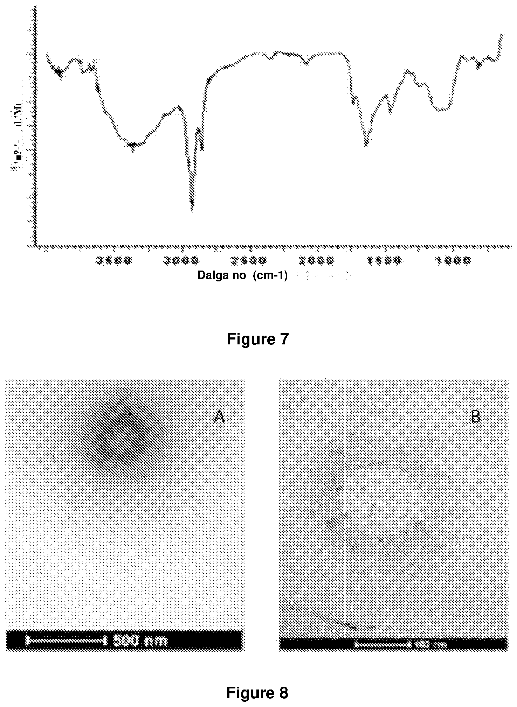

For this process, 2-10 mg of MNP-NH2; is mixed with sonication within PBS (pH: 7-8)containing 2-10 ml of polysorbate 20 (tween 20). Peptide-pemetrexed and peptide-pazopanib conjugatesare added to the medium such that the reaction medium has 5-15 mg of peptide-pemetrexed and 5-15 mg of peptide-pazopanib conjugates. Afterwards, by adding 0.05-0.2 mmol of N-ethyl-N'-(3-dimethylaminopropyl) carbodiimide hydrochloride (EDAC) and 0.1 - 0.4 mmol of N-hydroxysuccinimide (NHS), it is left to incubation under nitrogen gas at 20-30°C for 3-5 hours for completing the reaction. At the end of the duration, it is centrifuged at 7000-11000 rpm, magnetite (Fe₃O₄) -peptide-drug conjugates are precipitated, and supernatant is removed. The unreacted excess peptide-drug conjugations are washed with nanoparticulates PBS for removing EDAC and NHS from the medium and are removed from the medium by being centrifuged at 7000-11000 rpm. FTIR spectrum of the magnetic nanoparticulate conjugate (MNP-PPP) bonded to peptide-pemetrexed-pazopanib is given in Figure 7.

As the magnetic conjugate can only be separated from supernatant by precipitating and purged from impurities at high rpms, experiments in the invention are conducted at high speeds.

### FTIR Analysis

When chemical structure of the magnetic-peptide-drugs conjugate is examined, with large peaks in the 3300-3500cm⁻¹of wavelength area of N-H stretching peaks of the amine (-NH₂) groups available in structure of the drugs and peptide and the large peak in the 1000-1200 cm⁻¹ofarea of the C-N groups, the presence of these groups is verified. Aromatic ring C-H stretching vibration at 2950 cm⁻¹, in-ring C-C and C-H stretching vibrations at 1650 cm⁻¹ and 1639 cm⁻¹, available in the structure of drugs and peptide,were observed. CH stretching at 2940 cm⁻¹, O-H bending peak between 1480-1450 cm⁻¹, and also C = N peak expected to be between 2400-2100 cm⁻¹ at 2380 cm⁻¹ and 2090 cm⁻¹were seen at very weak intensity. That the 550 cm⁻¹of vibration band showing the structure as to Fe-O link available in the Fe₃-O₄molecule free from FTIR spectrum is observed at 600 cm⁻¹when it is bonded to the molecule structure was revealed in the magnetic nanoparticle structure synthesized by Lubambo et al. in 2015 (Lubambo et al., 2015). The drugs for the magnetic structure prove that the magnetic structure is bonded with the drugs and peptide in accord with the peak literature in 600 cm⁻¹of finger print region observed in FTIR spectrum of the nanoparticle synthesized with peptide and Fe₃-O₄.

### Synthesis of Nanobubbles Charged with Nanoconjugate

1,2-dipalmitoyl-sn-glycero-3-phosphocholine (DPPC), 1,2-dioleyl-sn-glycero-3-phosphocholine (DOPC), and cholesterol (CH) mixtures (5-10 mg DPPC, 5-10 mg DPOC, 5-10 mg CH) are dissolved in 2-8 mL of chloroform. Then, organic solvent is evaporated out by passing nitrogen gas in a water bath at the temperature of 40-60°C and this process is proceeded until a lipid film is formed around a vial wall. Afterwards, the entire solvent is left to rest for 4-6 hours under high pressure vacuum. At the end of the duration, the lipid film is left to rest for 10-20 minutes at room temperature with addition of nanoconjugate mixed in 0.3-0.4 M 1-5 ml of mannitol solution. Then, this mixture is left to incubation in sonic bath for 45-60 minutes and thus liposome formation is acquired. In an aim to obtain nano-size liposomes, polycarbonate membranes and extruder system having a pore diameter of 800 and 400 nanometers are used. For synthesis of nanobubbles, compressed nitrogen gas at 1-2 atm is passed through the liposomes for 15-45 minutes. Then, they are freezed on dry ice for 15-45 minutes. Freezed nanobubbles are taken from dry ice and left to dissolve to be at room temperature. The step of freezing and thawing is repeated for 3-5 times. The image of high contrast permeable electron microscope (C-TEM) of NB-800 (A) and NB-400 (B) is given in Figure 8.

### I CP-MS, Zeta Size and TEM Analysis

It was discovered that the hydrodynamic size of the bubble (NB-800) form that was passed through the membranes having a pore diameter of 800 nm was 491.1 nm; the hydrodynamic size of the bubble (NB-400) form that was passed through the membranes having a pore diameter of 400 nm was 275.8 nm. When considering the results, it can be concluded that NB-800 is suitable to be used in inhalation applications, on the other hand, NB-400 is suitable to be used in intravenous applications. That the nanobubbles obtained have a global shape is determined with reference to the TEM images of Figure 8.

**Table 1. Magnetite amount and encapsulation yields contained by nano-bubble structures.**

| | Microbubble | NB-800 | NB-400 nm |
|---|---|---|---|
| Magnetite Nanoparticle Concentration (µg/ml) | 77,07 | 24,30 | 14,00 |
| Encapsulation Yield % | 100 | 20,45 | 11,78 |

The nanoconjugate comprising magnetite can be encapsulated to the microbubble structure at a ratio of 100%. When nano is being formed liposome structures are opened and they are reformed in nano size. During this formation some part of the content is not encapsulated and the other part remains in the membrane. Due to this reason the encapsulation yield of MNP-PPP is reduced as the size is reduced.

### Cytotoxicity and Cell Viability Analysis

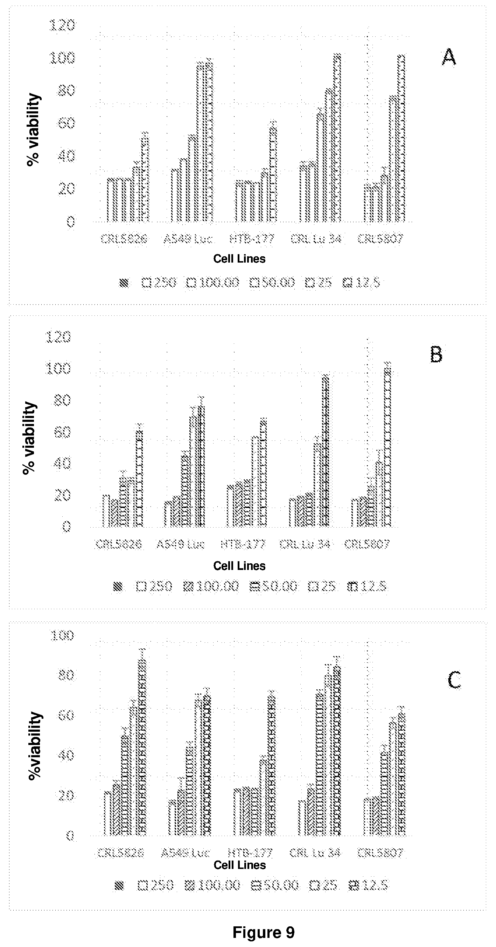

It has been noted that the samples of MNP-PPP, liposome (sieved through a 400 nm'lik pore diameter membrane) and bubble (sieved through a 400 nm'lik pore diameter membrane) created high rates of cytotoxicity with increasing concentration for all cells. It has been determined that a toxic effect has been formed in the CCD-34Lu cell which is a healthy cell similar to the effect formed in cancerous cells. It is believed that the damage that may be caused by the drug carrier in healthy cells can be changed in vivo. It has been noted that MNP-PPP samples (Figure 9-A) created 62-75%, liposome samples (Figure 9-B) created 73-83% and nanobubble samples (Figure 9-C) created 75-81% cytotoxic effect at aconcentration of 100 µg/mL for five cell lines after 48 hours.

The IC₅₀ values for 48 hours of the MNP-PPP sample has been recorded as 81,64±0,29 µg/mL for the A549-luc cell; 10,59±1,88 µg/mL for the HTB- 177 cell; 40,24±1,66 µg/mL for the CRL5807 cell; 7,09±0,79 µg/mL for the CRL5826 cell and 88,01±6,21 µg/mL for the CCD-34Lu cell.

The IC₅₀ values for 48 hours of the Liposome samples has been recorded as 38,56±0,81 µg/mL for the A549-luc cell; 28,28±0,74 µg/mL for the HTB- 177 cell; 28,09±0,60 µg/mL for the CRL5807 cell; 14,47±0,16 µg/mL for the CRL5826 cell and 29,42±1,00 µg/mL for the CCD-34Lu cell.

The IC₅₀ values for 48 hours of the NB-400 samples has been recorded as 40,59±0,84 µg/mL for the A549-luc cell; 19,99±0,64 µg/mL for the HTB-177 121 cell; CRL5807 hücresi için 27,03±3,09 µg/mL; CRL5826 hücresi için 48,46±0,47 µg/mL ve CCD-34Lu hücresi için 65,16±4,16 µg/mL'dir.

A brief period of ultrasound has been applied to cells following drug administration in order to see if the cytotoxicity of the NB-400 sample was altered or not. In the analyses carried out on the A549-luc cell as it shall be working in in vivo assays the IC₅₀ value on the 48^{th} hour of the NB-400 sample has been calculated as 40,99±2,62 µg/mL. When we look at the value if ultrasound was not applied, it has been noted that the IC50 value was decreased a little following ultrasound application (40,59±0,84 µg/mL for 48 hours; 34,89±1,75 µg/mL for 72 hours). Therefore it has been decided that nanobubble structures are more effective in releasing the drug inside their structure as being susceptible to ultrasound.

### Apoptosis Trials

The amounts of the cells at the live/necrotic/proapoptotic/apoptotic stages have been determined during the flow cytometry analysis carried out in order to understand the apoptosis effect of NB-400 on the A549-luc cells. It has been noted that the NB-400 sample at a concentration of 6,25 µg/ml had apoptotic effect at a ratio of 0,9% necrotic, 0,4% proapopotic and 0,1% apoptotic in comparion to the control group cells.

### Biyocompatibility Trials

As the plasma protein amount can vary from person to person, trials have been carried out in order to obtain nanobubble and plasma concentrations at ratios of 10:90, 20:80, 40:60, 60:40 (plazma:nanobubble). It has been noted that the serum protein bining amount increased depending on the increased amount of nanobubbles following the trial. The 60:40 ratio amount of binding has been determined as 44%

Haemolysis trials have been carried out by incubating the NB-400 sample with erythrocytes at different concentrations. Haemolysis has not been observed in comparison to the positive controls at other concentrations up to 250 µg/ml concentration of nanobubbles that have been added into the blood samples at varying ratios that have been adjusted to the 2% hematocrit rate. At a concentration of 250 µg/ml however a very low amount of haemolysis has been determined.

The cells have been incubated with nanobubbles for an hour in order to determined phagocytosis in macrophage cells of the NB-400 sample and at the end of the one hour period the morphological structures of the cells have been examined and their numbers have been determined. Accordingly, it has been noted that following the incubation of the cells with nanobubbles, the morphological structures of the cells was not altered however their numbers had been somewhat decreased. In order to determine the amount of NB-400 that was ingested into the cells, ICP-MS analysis has been used. The macrophage cells that were not administered drugs during analisys have been accepted as the control group and calculation has been made using the iron amount in cells that have been administered with drugs. It has been noted that 30,8% of the nanobubbles were received by the macrophage cells and therefore have been phagocytized. When all the data obtained is taken into consideration, it is believed that the nanobubbles are biocompatible.

### Biodistribution Trials

NB-400 which was formed by encapsulating FITC which has fluorescence effect has been injected to nude mice through their tail veins and after 30 minutes the distribution of the NB-400 sample inside the body was viewed using the in vivo imaging system (IVIS). Besides this NB-400 that was encapsulated with FITC was injected to another nude mouse and following this 30 minutes of magnet application was carried out at the tumor region and its image was captured. Following the magnet application ultrasound was applied to the tumor region of the mouse and images were taken by IVIS. It has been noted that when the magnet was not applied to the mouse the FITC sourced radiation was distributed to the entire body of the nude mouse. However following the magnet application the nanobubble structures accumulated at the area where the magnet was applied to as said structures contained magnetite. Besides this, following the magnet application, and following the ultrasound application, the scattering of radiation observed showed us that the nanobubbles had bursted by means of ultrasound and their contents had been released. After the images were taken, the nude mice were sacrificed under ethical conditions and their organs were collected in order to carry out an ICP-MS analysis in order to determine the nanoubble amount for Fe analysis. It has been determined that approximately 80% of the nanobubble strcutrure were accumulated inside the tumor found in the nude mice that were subjected to magnet application.

### Farmacokinetic Studies

NB-400 was given from the tail vein and following 3.min, 9.min., 30.min, 1.hr, 3.hrs, 9.hrs and 24.hrs the animals were sacrificed and Fe analysis was carried out in their organ, blood, urine and feces samples using ICP-MS and the % distribution in organs depending on time was calculated by means of the magnetic conjugate amounts found inside the prepared nanobubbles.

| | 3. min | 9. min | 30.min | 1.hour | 3.hours | 9.hours | 24.hours |
|---|---|---|---|---|---|---|---|
| Kidney | 19.86 | 31.24 | 36.13 | 61.68 | 23.68 | 7.12 | 7.09 |
| Liver | 28.26 | 20.07 | 24.23 | 20.23 | 19.4 | 11.02 | 4.35 |
| Lungs | 6.72 | 5.86 | 17.72 | 13.1 | 12.24 | 6.96 | 3.85 |
| Feces | 0 | 0 | 0 | 0 | 40.4 | 65.13 | 82.29 |
| Urine | 0 | 0 | 0 | 0 | 1.68 | 5.53 | 1.24 |
| Plasma | 45.25 | 43.83 | 20.97 | 10.68 | 3.83 | 0.83 | 0.57 |

### Treatment Applications

For intravenous treatment NB-400 has been injected through the tail vein and magnet has been applied to the tumor region for 30 minutes and after the magnet has been removed ultrasound has been applied.

It can be seen from the table values that the nanobubbles that have been prepared according to Table 2 have IV application treatment potential. When the tumor size is small treatment potential is higher.

**Table 2. Treatment results carried out with intravenous application of NB-400 through the tail vein**

| **Time** / **animal** | **1** | **2** | **3** | **4** | **5** | **6** |
|---|---|---|---|---|---|---|
| **Day** | 524,01 mm ³ | 340,64 mm³ | 564,45 mm³ | 634,7 mm³ | **338,4** mm³ | 444,29 mm³ |
| **7^{th} day** | - | - | - | 339,88 mm ³ | 2025,85 mm³ | 260,17 mm³ |
| **14^{th} day** | No tumors | 730,99 mm³ | No tumors | - | 2235,26 mm³ | No tumors |
| **21^{st} day** | No tumors | ex | No tumors | - | - | No tumors |

For inhaler treatment the NB-800 sample has been placed into the inhalation device and inhaler treatment has been applied to mice with lung cancer 3 times a week. Following this magnet has been applied to the tumor region for 30 minutes and when the magnet was removed ultrasound application was carried out.

As it can be seen from Table 3, it can be seen that there were alterations in the tumor sizes of the animals and different responses were given to treatment. It can be seen that treatment potential was high with the bubbles given by means of inhalation treatment if the tumor was small sized however when the tumor mass was higher it was perceived that treatment took longer even though it was successful. It can be seen that all of the animals within the group responded positively to treatment.

**Table 3. Treatment results carried out with the NB-800 inhaler application**

| **Time/ Animal** | **1** | **2** | **3** | **4** | **5** |
|---|---|---|---|---|---|
| **0 Days** | 771,0 mm ³ | 223,90 mm ³ | 1745,33 mm ³ | 634,7 mm ³ | 709,11 mm ³ |
| **5^{th} day** | - | - | - | 600,97 mm³ | No Tumors |
| **9^{th} day** | 657,88 mm ³ | - | - | No Tumors | No Tumors |
| **10^{th} day** | - | No Tumors | - | No Tumors | No Tumors |
| **12^{th} day** | - | No Tumors | 1358,43 mm³ | - | - |
| **15^{th} day** | - | No Tumors | 1230,0 mm³ | - | - |
| **19^{th} day** | 407,48 mm³ | No Tumors | | - | - |
| **26^{th} day** | 360,98 mm ³ | - | - | - | - |

### Toxicology Studies

### Single Dose Toxicity Test

According to the "up and down" test carried out in relation to the "OECD Guideline Number 425", the test agent did not show acute toxic effects at 2.54mg/ml test concentrations.

### Salmonella/Microsome (AMES) Test

It has been determined that the test agent did not have any kind of effect that may lead to mutation on the Salmonella typhimurium TA 98 andTA 100 strains at the tested concentrations.

### 28 Day Repeat Dose Toxicity Test

0,7-1,0 ml blood samples were obtained from nearly all mice under ketamine+xylazine anesthesia after the completion of the 28 day repeat dose toxicity trial by inhaler and IV applications and haemogram analysis was carried out by outsourced support. During the IV and inhaler application toxicology study, it has been observed that the blood parameter values were between reference ranges and deviation from the reference values were not observed during the haemogram analysis at 3 different doses of the preparation.

### Ex vivo Studies

Immunohistochemical studies have shown that the apoptosis amount in the tumor tissue belonging to bubble formulations applied to mice were higher in comparison to the control groups and that the VEGF expression was lower.

## Claims

1. An ultrasound susceptible magnetic directed nano drug carrier system preparation method, **characterized by** comprising the following process steps;
i) Synthesizing the magnetic nanoparticles (MNP), preferably by means of the coprecipitation method
ii) Amination of the synthesized magnetic nanoparticles,
iii) Modifying drugs, where drugs being pemetrexed and pazopanib
iv) Conjugating each of the modified drugs with different peptides,
v) Obtaining nanoconjugates by binding the peptide-drug conjugates to the aminated magnetic nanoparticles,
vi) Synthesizing the nanoconjugate loaded nanobubbles.

2. A method according to claim 1, **characterized by** comprising the following process steps when synthesizing magnetic nanoparticles,
a) Dissolving of 0.5-2 mol iron (II) chloride (FeCl₂) and 1-4 mol iron (III) chloride (FeCl₃) inside deoxidized pure water at a 300-S000rpm mixing speed in a fixed temperature jacketed reactor,
b) Adjusting the pH medium to have an alkali medium by adding concentrated base solution as a precipitation agent into the solution,
c) Leaving the solution to be incubated for 1-3 hours in order to obtain nucleation under fixed temperature and vigorous mixing conditions,
d) Separating the nanoparticles that have been formed following incubation by means of magnetic decantation,
e) Washing the nanoparticles with distilled water and ethanol (EtOH),
f) Drying the washed nanoparticles in an incubator.

3. A method according to claim 1, **characterized by** the process of aminating the synthesized magnetic nanoparticles comprises the steps of;
a) Adding 2-aminoetthylphosphonic acid (APA) solution into the medium having a pH of 5-7 and containing the magnetic nanoparticles synthesized during the process step (i),
b) Leaving the solution to be incubated at a temperature of 70-90°C and at 1500-3000 rpm for reaction,
c) Preparing the magnetic nanoparticles (MNP- NH₂) that carry active amine groups following incubation, to be distributed inside a phosphate buffered saline (PBS) by being washed with water with the aid of magnetic decantation.

4. A method according to claim 1, **characterized in that** the modification of pemetrexed in the process step (iii) comprises the process steps of;
a) Slowly adding 100-200 mmol thionyl chloride (SOCl₂) into cold 5-15 ml methanole (MeOH) at -15-(-5)°C,
b) Leaving the solution for reaction at -10-(-5)°C for 15-35 minutes,
c) Following reaction, adding 50-150 mg pemetrexedin sodium salt to the solution and bringing the temperature of the reaction medium to 20-30°C and incubating for 2-4 hours by mixing under a condenser,
d) Separating the sodium chloride (NaCl) obtained following reaction by filtering,
e) Evaporating the methonol (MeOH) by vacuum,
f) Removing the thionyl chloride (SOCl₂) compound by successively dissolving it in extremely hot methanol,
g) Crystallizing the obtained product in ethanol,
h) Obtaining methoxylated pemetrexed (Pem(OMe)₂₎ crystals by removing the obtained crystals with a filter and treating them by diethylether ((C₂H₅)₂0) and drying them,
i) Dissolving the methoxylated pemetrexed (Pem(OMe)2) crystals inside 0,3-0,5 ml dimethyl formamide (DMF),
j) Adding 7-15 µl diisopropylethylamine and 0,10-0,20 mg chloroacetic anhydride into the solution and leaving it to react for 4-6 hours at a temperature of 30-50°C,
k) Adding acetonitrile (ACN): distilled water at a ratio of 80:20%-70:30% (v/v) comprising 0,05-0,15 trifluoroacetic acid (TFA) and 0,1-0,3 ml distilled water into the mixture at the end of the reaction period and leaving it for incubation at room temperature for 10-20 hours,
l) Sedimenting the product by adding distilled water into the medium following incubation,
m) Removing the product from the medium by means of centrifugation,
n) Obtaining chloroacetylated pemetrexed (CIAc-Pem(OMe₂)) crystals by leaving the product to dry after it has been treated with diethyl ether and crystallized in ethanol.

5. A method according to claim 4, **characterized by** modification being applied in order to chloroacetylate the pazopanib during the process step (iii).

6. A method according to claim 4, **characterized in that** the modification of pazopanib during the process step (iii) comprises the process steps of;
a) Dissolving the pazopanib in 0,3-0,5 ml dimethyl formamide (DMF),
b) Adding 7-15 µl diisopropylethylamine and 0,10-0,20 mg chloroacetic anhydrite into the solution and leaving it to react at 30-50°C temperature for 4-6 hours,
c) Adding acetonitrile (ACN): distilled water at a ratio of 80:20%-70:30% (v/v) comprising 0,05-0,15 trifluoroacetic acid (TFA) and 0,1-0,3 ml distilled water into the mixture at the end of the reaction period and leaving it for incubation at room temperature for 10-20 hours,
d) Sedimenting the product by adding distilled water into the medium following incubation,
e) Removing the product from the medium by means of centrifugation,
f) Obtaining chloroacetylated pazopanib crystals by leaving the product to dry after it has been treated with diethyl ether and crystallized in ethanol.

7. A method according to claim 1, **characterized in that** the conjugation of each of the modified drugs with different peptites, in the process step (iv) comprises the process steps of;
a) Dissolving the 0,01-0,05 mmol modified drug inside 7-15 ml dimethylformamide (DMF),
b) Adding 5-15 ml 0,05-0,2 M tris buffer (pH: 7-8) to the solution,
c) Adding 0,005-0,025 mmol peptide slowly into the reaction medium,
d) Leaving the peptide-drug mixture for incubation for 15-25 hours in an agitation incubator at room temperature,
e) Obtaining peptide-drug conjugates by removing the solution following incubation.

8. A method according to claim 7, **characterized in that** the pemetrexed and pazopanib that have been modified in the process step (iv) have each been conjugated with separate peptides.

9. A method according to claim 1, **characterized by** the mentioned peptide structure being Ala-Lys-Ala-Leu-Arg- Cys.

10. A method according to claim 1, **characterized in that** obtaining nanoconjugates in the process step (v) comprises the process steps of;
a) Mixing the magnetic nanoparticles (MNP-NH₂) carrying a 2-10 mg active amine group inside PBS (pH:7-8) containing 2-10 ml polysorbate 20 (tween 20) by sonication,
b) Adding 5-15 mg pemetrexed-peptide and 5-15 mg pazopanib-peptide conjugates into the medium,
c) Leaving the mixture for incubation under nitrogen gas at 20-30°C for 3-5 hours in order to complete the reaction by adding 0,05-0,2 mmol N-ethyl-N'-(3-dimethylamino propyl) carbodiimidehydrochloride (EDAC) and 0,1-0,4 mmol N-hydroxysuccinimide (NHS) into the mixture,
d) Centrifuging the mixture at 7000-11000 rpm following incubation, sedimenting the magnetite (Fe₃0₄)- peptide-drug conjugates and separating the supernatant,
e) Washing the nanoconjugates with PBS in order to remove EDAC and NHS and the inert severe peptide-drug conjugates from the medium and centrifuging at 7000-11000 rpm to remove them from the medium.

11. A method according to claim 1, **characterized in that** the synthesis of nanoconjugate loaded nanobubbles in the process step (vi) comprises the process steps of;
a) Dissolving the mixture of 5-10 mg 1,2- dipalmitoyl-sn-glycero-3-phosphocholine (DPPC), 5-10 mg 1,2-dioleyl-sn-glycero- 3-phosphocholine (DOPC) and 5-10 mg cholesterol (CH) inside a 2-8 mL organik solution, preferably inside chloroform,
b) Evaoprating the solution in a water bath at a temperature of 40-60°C by passing argon gas (Ar) until a lipid film has been formed,
c) Leaving the mixture to rest for 4-6 hours until all of the solution is evaporated under high pressure vacuum,
d) Leaving the mixture to rest at room temperature for 10-20 minutes by adding nanoconjugate mixed inside the 0,3-0,4 M 1-5 ml mannitol solution in lipid film at the end of the evaporation period,
e) Ensuring liposome is formed by leaving the mixture to be incubated at a sonic bath for 45-60 minutes,
f) Extruding liposome comprising nanoconjugates, in the extruder system and polycarbonate membranes having an 800-400 nanometer pore diameters in order to obtain nano sized liposomes,
g) forming nanobubbles by passing argon gas (Ar) with 1-2 atm pressure over the liposomes that are nano sized for 15-45 minutes,
h) Freezing the nanobubbles in dry ice for 15-45 minutes,
i) Taking out the frozen nanobubbles and leaving them to thaw until they reach room temperature.

## Patentansprüche

1. Ein ultraschallempfindliches, magnetisch gesteuertes Verfahren zur Herstellung eines Nano-Medikamentträgersystems, **dadurch gekennzeichnet, dass** es die folgenden Verfahrensschritte umfasst;
i) Synthese der magnetischen Nanopartikel (MNP), vorzugsweise mit Hilfe der Kopräzipitationsmethode
ii) Aminierung der synthetisierten magnetischen Nanopartikel,
die Medikamente Pemetrexed und Pazopanib sind
iv) Konjugieren der modifizierten Medikamente mit verschiedenen Peptiden,
v) Gewinnung von Nanokonjugaten durch Bindung der Peptid-Medikament-Konjugate an die aminierten magnetischen Nanopartikel,
vi) Synthese der mit Nanokonjugaten beladenen Nanoblasen.

2. Ein Verfahren nach Anspruch 1, **dadurch gekennzeichnet, dass** es bei der Synthese von magnetischen Nanopartikeln die folgenden Verfahrensschritte umfasst,
a) Auflösen von 0,5-2 Mol Eisen(II)-chlorid (FeCl2) und 1-4 Mol Eisen(III)-chlorid (FeCI₃) in desoxidiertem reinem Wasser bei einer Mischgeschwindigkeit von 300-S000 U./min. in einem Mantelreaktor mit fester Temperatur,
b) Einstellen des pH-Wertes des Mediums auf ein alkalisches Medium durch Zugabe von konzentrierter Basenlösung als Fällungsmittel in die Lösung,
c) Inkubieren der Lösung für 1 bis 3 Stunden, um eine Keimbildung bei fester Temperatur und kräftigem Mischen zu erreichen,
d) Abtrennung der nach der Inkubation gebildeten Nanopartikel durch magnetische Dekantierung,
e) Waschen der Nanopartikel mit destilliertem Wasser und Ethanol (EtOH),
f) Trocknen der gewaschenen Nanopartikel in einem Inkubator.

3. Ein Verfahren nach Anspruch 1, **dadurch gekennzeichnet, dass** das Verfahren zum Aminieren der synthetisierten magnetischen Nanopartikel die folgenden Schritte umfasst;
a) Zugabe von 2-Aminoethylphosphonsäure (APA)-Lösung in das Medium mit einem pH-Wert von 5-7, das die im Verfahrensschritt (i) synthetisierten magnetischen Nanopartikel enthält,
b) Inkubation der Lösung bei einer Temperatur von 70-90°C und 1500-3000 U./min., um ein Reaktion zu erhalten,
c) Vorbereitung der magnetischen Nanopartikel (MNP-NH₂), die nach der Inkubation aktive Amingruppen tragen, um sie in einer phosphatgepufferten Salzlösung (PBS) zu verteilen, indem sie mit Hilfe einer magnetischen Dekantierung mit Wasser gewaschen werden.

4. Ein Verfahren nach Anspruch 1, **dadurch gekennzeichnet, dass** die Modifikation von Pemetrexed im Verfahrensschritt (iii) die folgenden Verfahrensschritte umfasst;
a) Langsame Zugabe von 100-200 mmol Thionylchlorid (SOCl₂) in 5-15 ml kaltes Methanol (MeOH) bei -15-(-5) °C,
b) Belassen der Lösung für 15-35 Minuten bei -10-(-5)°C, um ein Reaktion zu erhalten,
c) Nach der Reaktion 50-150 mg Pemetrexedin-Natriumsalz zu der Lösung dazugeben, die Temperatur des Reaktionsmediums auf 20-30 °C bringen und es 2-4 Stunden lang unter Rühren unter einem Kühler inkubieren,
d) Nach der Reaktion erhaltene Natriumchlorid (NaCI) durch Filtrieren trennen,
e) Verdampfen des Methonols (MeOH) im Vakuum,
f) Entfernen der Thionylchlorid-Verbindung (SOCl₂) durch sukzessives Auflösen in extrem heißem Methanol,
g) Auskristallisieren des erhaltenen Produkts in Ethanol,
h) Gewinnen der methoxylierten (Pem(OMe)₂)Pemetrexed-Kristalle, indem die erhaltenen Kristalle mit einem Filter entfernt werden und mit Diethylether ((C₂H₅)₂O) behandelt und getrocknet werden,
i) Lösen der methoxylierter (Pem(OMe)₂₎ Pemetrexed-Kristalle in 0,3-0,5 ml Dimethylformamid (DMF),
j) Zugabe von 7-15 µl Diisopropylethylamin und 0,10-0,20 mg Chloressigsäureanhydrid in die Lösung und die Lösung 4-6 Stunden lang bei einer Temperatur von 30-50°C reagieren lassen,
k) Zugabe von Acetonitril (ACN): destilliertem Wasser im Verhältnis 80:20%-70:30% (v/v) mit 0,05-0,15 Trifluoressigsäure (TFA) und 0,1-0,3 ml destilliertem Wasser in die Mischung am Ende der Reaktionszeit und Inkubieren bei Raumtemperatur für 10-20 Stunden,
l) Sedimentieren des Produkts durch Zugabe von destilliertem Wasser in das Medium nach der Inkubation,
m) Entfernen des Produkts aus dem Medium durch Zentrifugieren,
n) Gewinnen von chloracetylierten (CIAc-Pem(OMe₂)) Pemetrexed-Kristallen, indem man das Produkt trocknen lässt, nachdem es mit Diethylether behandelt und in Ethanol kristallisiert wurde.

5. Ein Verfahren nach Anspruch 4, **dadurch gekennzeichnet, dass** die Modifikation angewendet wird, um das Pazopanib während des Verfahrensschritts (iii) zu chloracetylieren.

6. Ein Verfahren nach Anspruch 4, **dadurch gekennzeichnet, dass** die Modifikation von Pazopanib während des Verfahrensschritts (iii) die folgenden Verfahrensschritte umfasst;
a) Lösen des Pazopanibs in 0,3-0,5 ml Dimethylformamid (DMF),
b) Zugabe von 7-15 µl Diisopropylethylamin und 0,10-0,20 mg Chloressigsäureanhydrit in die Lösung und die Lösung bei 30-50°C Temperatur für 4-6 Stunden reagieren lassen,
c) Zugabe von Acetonitril (ACN): destilliertem Wasser im Verhältnis 80:20%-70:30% (v/v) mit 0,05-0,15 Trifluoressigsäure (TFA) und 0,1-0,3 ml destilliertem Wasser in die Mischung am Ende der Reaktionszeit und Inkubieren bei Raumtemperatur für 10-20 Stunden,
d) Sedimentieren des Produkts durch Zugabe von destilliertem Wasser in das Medium nach der Inkubation,
e) Entfernen des Produkts aus dem Medium durch Zentrifugieren,
f) Gewinnen von chloracetylierten Pazopanib-Kristallen, indem man das Produkt trocknen lässt, nachdem es mit Diethylether behandelt und in Ethanol kristallisiert wurde.

7. Ein Verfahren nach Anspruch 1, **dadurch gekennzeichnet, dass** die Konjugation jedes der modifizierten Medikamente mit verschiedenen Peptiden im Verfahrensschritt (iv) die folgenden Verfahrensschritte umfasst;
a) Auflösen des mit 0,01-0,05 mmol modifizierten Medikaments in 7-15 ml Dimethylformamid (DMF),
b) Zugabe von 5-15 ml 0,05-0,2 M Trispuffer (pH: 7-8) zu der Lösung,
c) Langsame Zugabe von 0,005-0,025 mmol Peptid in das Reaktionsmedium,
d) Die Peptid-Wirkstoff-Mischung wird 15-25 Stunden lang in einem Rührinkubator bei Raumtemperatur inkubiert,
e) Gewinnung von Peptid-Medikament-Konjugaten durch Entfernen der Lösung nach der Inkubation.

8. Ein Verfahren nach Anspruch 7, **dadurch gekennzeichnet, dass** das Pemetrexed und Pazopanib, die im Verfahrensschritt (iv) modifiziert wurden, jeweils mit separaten Peptiden konjugiert wurden.

9. Ein Verfahren nach Anspruch 1, **dadurch gekennzeichnet, dass** die genannte Peptidstruktur Ala-Lys-Ala-Leu-Arg-Cys ist.

10. Ein Verfahren nach Anspruch 1, **dadurch gekennzeichnet, dass** die Herstellung von Nanokonjugaten im Verfahrensschritt (v) die folgenden Verfahrensschritte umfasst;
a) Mischen durch Beschallung der magnetischen Nanopartikel (MNP-NH₂), die 2-10 mg aktive Amingruppen tragen, in PBS (pH: 7-8), das 2-10 ml Polysorbat 20 (Tween 20) enthält,
b) Zugabe von 5-15 mg Pemetrexed-Peptid- und 5-15 mg Pazopanib-Peptid-Konjugaten in das Medium,
c) Inkubation der Mischung unter Stickstoffgas bei 20-30°C für 3-5 Stunden, um die Reaktion zu vervollständigen, indem 0,05-0,2 mmol N-Ethyl-N'-(3-dimethylamino propyl) carbodiimidehydrochlorid (EDAC) und 0,1-0,4 mmol N-Hydroxysuccinimid (NHS) in die Mischung gegeben werden,
d) Zentrifugieren der Mischung bei 7000-11000 U./min. nach der Inkubation, Absetzen der Magnetit (Fe₃O₄)-Peptid-Medikament-Konjugate und Abtrennen des Überstands,
e) Waschen der Nanokonjugate mit PBS, um EDAC und NHS sowie die inerten schweren Peptid-Medikament-Konjugate aus dem Medium zu entfernen, und Zentrifugieren bei 7000-11000 U./min., um sie aus dem Medium zu entfernen.

11. Ein Verfahren nach Anspruch 1, **dadurch gekennzeichnet, dass** die Synthese von mit Nanokonjugaten beladenen Nanoblasen im Verfahrensschritt (vi) die folgenden Verfahrensschritte umfasst;
a) Auflösen der Mischung aus 5-10 mg 1,2-Dipalmitoyl-sn-glycero-3-phosphocholin (DPPC), 5-10 mg 1,2-Dioleyl-sn-glycero-3-phosphocholin (DOPC) und 5-10 mg Cholesterin (CH) in einer 2-8 mL organischen Lösung, vorzugsweise in Chloroform,
b) Die Lösung in einem Wasserbad bei einer Temperatur von 40-60°C durch Einleiten von Argongas (Ar) verdampfen, bis sich ein Lipidfilm gebildet hat,
c) Das Gemisch 4-6 Stunden ruhen lassen, bis die gesamte Lösung unter Hochdruckvakuum verdampft ist,
d) Das Gemisch 10-20 Minuten bei Raumtemperatur ruhen lassen, indem das Nanokonjugat am Ende der Verdampfungszeit in die 0,3-0,4 M 1-5 ml Mannitol-Lösung im Lipidfilm eingerührt wird,
e) Sicherstellen, dass sich Liposomen bilden, indem die Mischung im Schallbad
für 45-60 Minuten inkubiert wird,
f) Extrudieren von Liposomen, die Nanokonjugate enthalten, im Extrudersystem und Polycarbonatmembranen mit einem Porendurchmesser von 800-400 Nanometern, um Liposomen in Nanogröße zu erhalten,
g) Bilden von Nanoblasen, indem Argongas (Ar) mit einem Druck von 1-2 atm für 15-45 Minuten über die Liposomen in Nanogröße geleitet wird,
h) Einfrieren der Nanoblasen in Trockeneis für 15-45 Minuten,
i) Entnahme der gefrorenen Nanoblasen und Auftauen lassen, bis sie Raumtemperatur erreicht haben.

## Revendications

1. Procédé de préparation de système de vecteur de nanomédicament à orientation magnétique sensible aux ultrasons, **caractérisé en ce qu'**il comprend les étapes de processus suivantes ;
i) La synthèse des nanoparticules magnétiques (MNP), de préférence au moyen du procédé de coprécipitation
ii) L'amination des nanoparticules magnétiques synthétisées,
iii) La modification des médicaments, où les médicaments sont le pémétrexed et le pazopanib
iv) La conjugaison de chacun des médicaments modifiés avec différents peptides,
v) L'obtention de nanoconjugués en liant les conjugués peptide-médicament aux nanoparticules magnétiques aminées,
vi) La synthèse des nanobulles chargées de nanoconjugués.

2. Procédé selon la revendication 1, **caractérisé en ce qu'**il comprend les étapes de processus suivantes lors de la synthèse des nanoparticules magnétiques,
a) La dissolution de 0,5-2 mol de chlorure de fer (II) (FeCl2) et de 1-4 mol de chlorure de fer (III) (FeCl₃) dans de l'eau pure désoxydée à une vitesse de mélange de 300-S000 tours/minute dans un réacteur à double enveloppe à température fixe,
b) L'ajustement du milieu du pH pour avoir un milieu alcalin en ajoutant une solution de base concentrée en tant qu'agent de précipitation dans la solution,
c) L'incubation de la solution pendant 1-3 heures afin d'obtenir une nucléation sous une température fixe et des conditions de mélange vigoureuses,
d) La séparation des nanoparticules qui ont été formées après l'incubation au moyen d'une décantation magnétique,
e) Le lavage des nanoparticules avec de l'eau distillée et de l'éthanol (EtOH),
f) Le séchage des nanoparticules lavées dans un incubateur.

3. Procédé selon la revendication 1, **caractérisé en ce que** le processus d'amination des nanoparticules magnétiques synthétisées comprend les étapes consistant à ;
a) Ajouter une solution d'acide 2-aminoéthylphosphonique (APA) dans le milieu ayant un pH de 5-7 et contenant les nanoparticules magnétiques synthétisées au cours de l'étape de processus (i),
b) Laisser la solution incuber à une température de 70-90 °C et à 1 500-3 000 tours/minute pour la réaction,
c) Préparer les nanoparticules magnétiques (MNP-NH₂) qui portent des groupes amines actifs après l'incubation, à être distribuées dans une solution saline tamponnée au phosphate (PBS) en étant lavées avec de l'eau à l'aide de la décantation magnétique.

4. Procédé selon la revendication 1, **caractérisé en ce que** la modification du pémétrexed dans l'étape de processus (iii) comprend les étapes de processus consistant à ;
a) Ajouter lentement 100-200 mmol de chlorure de thionyle (SOCl₂) dans 5-15 ml de méthanol (MeOH) froid à -15- (-5) °C,
b) Laisser la solution pour la réaction à -10-(-5) °C pendant 15-35 minutes,
c) Après la réaction, ajouter 50-150 mg de pémétrexed en sel de sodium à la solution et porter la température du milieu réactionnel à 20-30 °C et incuber pendant 2-4 heures en mélangeant sous un condensateur,
d) Séparer le chlorure de sodium (NaCl) obtenu après la réaction par filtrage,
e) Faire évaporer le méthanol (MeOH) sous vide,
f) Éliminer le composé de chlorure de thionyle (SOCl₂) en le dissolvant successivement dans du méthanol extrêmement chaud,
g) Cristalliser le produit obtenu dans l'éthanol,
h) Obtenir des cristaux de pémétrexed méthoxylés (Pem(OMe)₂₎ en éliminant les cristaux obtenus avec un filtre et en les traitant avec de l'éther diéthylique ((C₂H₅)₂O) et en les séchant,
i) Dissoudre les cristaux de pémétrexed méthoxylés (Pem(OMe)₂₎ dans 0,3-0,5 ml de diméthylformamide (DMF),
j) Ajouter 7-15 µI de diisopropyléthylamine et 0,10-0,20 mg d'anhydride chloracétique à la solution et la laisser réagir pendant 4-6 heures à une température de 30-50 °C,
k) Ajouter de l'acétonitrile (ACN) : de l'eau distillée dans un rapport de 80:20 %-70:30 % (v/v) comprenant 0,05-0,15 d'acide trifluoroacétique (TFA) et 0,1-0,3 ml d'eau distillée dans le mélange à la fin de la période de réaction et le laisser incuber à température ambiante pendant 10-20 heures,
l) Sédimenter le produit en ajoutant de l'eau distillée dans le milieu après l'incubation,
m) Éliminer le produit du milieu au moyen de la centrifugation,
n) Obtenir des cristaux de pémétrexed chloracétylés (CIAc-Pem(OMe₂)) en laissant sécher le produit après qu'il a été traité avec de l'éther diéthylique et cristallisé dans l'éthanol.

5. Procédé selon la revendication 4, **caractérisé en ce que** la modification est appliquée afin de chloroacétyler le pazopanib au cours de l'étape de processus (iii).

6. Procédé selon la revendication 4, **caractérisé en ce que** la modification du pazopanib au cours de l'étape de processus (iii) comprend les étapes de processus consistant à ;
a) Dissoudre le pazopanib dans 0,3-0,5 ml de diméthylformamide (DMF),
b) Ajouter 7-15 µl de diisopropyléthylamine et 0,10-0,20 mg d'anhydrite chloroacétique à la solution et la laisser réagir à une température de 30-50 °C pendant 4-6 heures,
c) Ajouter de l'acétonitrile (ACN) : de l'eau distillée dans un rapport de 80:20 %-70:30 % (v/v) comprenant 0,05-0,15 d'acide trifluoroacétique (TFA) et 0,1-0,3 ml d'eau distillée dans le mélange à la fin de la période de réaction et le laisser incuber à température ambiante pendant 10-20 heures,
d) Sédimenter le produit en ajoutant de l'eau distillée dans le milieu après l'incubation,
e) Éliminer le produit du milieu au moyen de la centrifugation,
f) Obtenir des cristaux de pazopanib chloroacétylés en laissant sécher le produit après qu'il a été traité avec de l'éther diéthylique et cristallisé dans l'éthanol.

7. Procédé selon la revendication 1, **caractérisé en ce que** la conjugaison de chacun parmi les médicaments modifiés avec différents peptides, dans l'étape de processus (iv), comprend les étapes de processus consistant à ;
a) Dissoudre le médicament modifié de 0,01-0,05 mmol dans 7-15 ml de diméthylformamide (DMF),
b) Ajouter 5-15 ml de tampon Tris 0,05-0,2 M (pH : 7-8) à la solution,
c) Ajouter lentement 0,005-0,025 mmol de peptide dans le milieu réactionnel,
d) Laisser le mélange peptide-médicament incuber pendant 15 à 25 heures dans un incubateur à agitation à température ambiante,
e) Obtenir des conjugués peptide-médicament en éliminant la solution après l'incubation.

8. Procédé selon la revendication 7, **caractérisé en ce que** le pémétrexed et le pazopanib qui ont été modifiés au cours l'étape de processus (iv) ont chacun été conjugués avec des peptides distincts.

9. Procédé selon la revendication 1, **caractérisé en ce que** la structure peptidique mentionnée est Ala-Lys-Ala-Leu-Arg-Cys.

10. Procédé selon la revendication 1, **caractérisé en ce que** l'obtention de nanoconjugués dans l'étape de processus (v) comprend les étapes de processus consistant à ;
a) Mélanger les nanoparticules magnétiques (MNP-NH₂) portant un groupe amine actif de 2-10 mg dans un PBS (pH : 7-8) contenant 2-10 ml de polysorbate 20 (tween 20) par sonication,
b) Ajouter 5-15 mg de conjugués pémétrexed-peptide et 5-15 mg de conjugués pazopanib-peptide dans le milieu,
c) Laisser le mélange incuber sous azote gazeux à 20-30 °C pendant 3-5 heures afin de terminer la réaction en ajoutant 0,05-0,2 mmol de N-éthyl-N'-(3-diméthylamino propyl) chlorhydrate de carbodiimide (EDAC) et 0,1-0,4 mmol de N-hydroxysuccinimide (NHS) dans le mélange,
d) Centrifuger le mélange à 7 000-11 000 tours/minute après l'incubation, sédimenter les conjugués (Fe₃O₄)-peptide-médicament de magnétite et séparer le surnageant,
e) Laver les nanoconjugués avec du PBS afin d'éliminer l'EDAC et le NHS ainsi que les conjugués peptide-médicament sévères inertes du milieu et centrifuger à 7 000-11 000 tours/minute pour les éliminer du milieu.

11. Procédé selon la revendication 1, **caractérisé en ce que** la synthèse des nanobulles chargées de nanoconjugués dans l'étape de processus (vi) comprend les étapes de processus consistant à ;
a) Dissoudre le mélange de 5-10 mg de 1,2-dipalmitoyl-sn-glycéro-3-phosphocholine (DPPC), 5-10 mg de 1,2-dioleyl-sn-glycéro-3-phosphocholine (DOPC) et 5-10 mg de cholestérol (CH) dans une solution organique de 2-8 ml, de préférence dans du chloroforme,
b) Évaporer la solution dans un bain-marie à une température de 40-60 °C en faisant passer du gaz argon (Ar) jusqu'à ce qu'un film lipidique se forme,
c) Laisser le mélange reposer pendant 4-6 heures jusqu'à ce que toute la solution soit évaporée sous vide à haute pression,
d) Laisser le mélange reposer à température ambiante pendant 10-20 minutes en ajoutant le nanoconjugué mélangé dans la solution de mannitol 0,3-0,4 M 1-5 ml dans le film lipidique à la fin de la période d'évaporation,
e) S'assurer de la formation du liposome en laissant le mélange incuber dans un bain sonique pendant 45-60 minutes,
f) Extruder le liposome comprenant les nanoconjugués, dans le système d'extrusion et les membranes en polycarbonate ayant un diamètre de pore de 800-400 nanomètres afin d'obtenir des liposomes de taille nanométrique,
g) former des nanobulles en faisant passer du gaz argon (Ar) avec une pression de 1-2 atm sur les liposomes qui sont de taille nanométrique pendant 15-45 minutes,
h) Congeler les nanobulles dans de la glace sèche pendant 15-45 minutes,
i) Retirer les nanobulles congelées et les laisser décongeler jusqu'à ce qu'elles atteignent la température ambiante.
